# EUROPEAN PATENT SPECIFICATION

(11) **EP 1 584 407 B1**
(45) Date of publication and mention of the grant of the patent: **02.11.2016**
(21) Application number: 05252151.5
(22) Date of filing: 06.04.2005
(51) Int. Cl.: B23K 35/00, B23K 35/32, B23K 103/24

(54) **Bonding titanium to stainless steel**
Verbinden von Titan mit rostfreiem Sahl
Liaison entre titane et acier inoxydable

(30) Priority: 07.04.2004 US 821023; 14.04.2004 US 823963; 27.04.2004 US 833588
(43) Date of publication of application: 12.10.2005
(73) Proprietor: The Alfred E Mann Foundation for Scientific Research, Santa Clarita, CA 91380-9005 (US)
(72) Inventor: Antalfy Attila, Bristol BS6 7TF (GB); Jiang Guangqiang, Irvine CA 92602 (US); Schnittgrund, Gary, D, Coeur d'Alene ID 83814 (US)
(74) Representative: Carter, Stephen John

(56) References cited:
- FR-A- 2 233 128
- GB-A- 746 648
- GB-A- 1 430 587
- US-A1- 2002 192 481
- US-B1- 6 221 513
- PATENT ABSTRACTS OF JAPAN vol. 018, no. 054 (M-1549), 27 January 1994 (1994-01-27) & JP 05 277759 A (POWER REACTOR & NUCLEAR FUEL DEV CORP; others: 01), 26 October 1993 (1993-10-26)

## Description

### FIELD OF THE INVENTION

The present invention relates to methods for bonding titanium to stainless steel, stainless steel / titanium component assemblies and to implantable miniature titanium to stainless steel connectors.

### INTRODUCTION

FR 2233128 describes a method for a brazed connection, more particularly heat treatment for heating the area involved in brazing between a titanium alloy and a stainless steel.

JP5277759A describes a method for producing high strength, high corrosion-resistant heterogeneous metal pipe joints.

GB 1430587 A describes diffusion bonding, which may be considered brazing. This document further describes the use of a metallic interlayer and a transient liquid phase between the parts being joined to bond different metals.

### SUMMARY OF THE INVENTION

The invention provides a component assembly suitable for use in a living tissue as set forth in claim 1, methods of bonding a stainless steel and titanium component as set forth in claim 12, , and a brazed component assembly as set forth in claim 25.

### BRIEF DESCRIPTION OF THE DRAWINGS

Embodiments of various aspects of the invention are described below, by way of example, with reference to the accompanying drawings, in which:
**FIG. 1** illustrates a side view of the component assembly with filler material as a foil between the stainless steel part and the titanium part.
**FIG. 2** schematically depicts the bonding steps of the present invention.
**FIG. 3** presents an isometric view of a titanium-nickel laminated filler material having three foil layers.
**FIG. 4** presents an isometric view of a titanium-nickel laminated filler material having five foil layers.
**FIG. 5** illustrates the compact filler material comprised of discrete particles of titanium and nickel.
**FIG. 6** presents a cross-sectional view of a discrete particle of nickel and titanium layers.
**FIG. 7** presents an exploded isometric view of a ceramic tube, titanium part, and stainless part.
**FIG. 8** illustrates a bonded device with a crimp attached wire.

### DETAILED DESCRIPTION OF THE PREFERRED EMBODIMENT

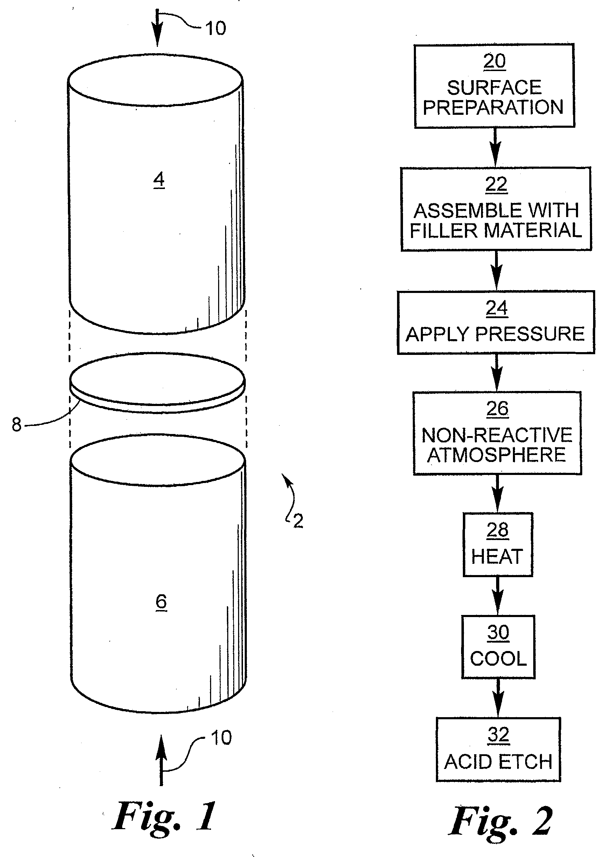

**FIG. 1** presents component assembly 2 having a titanium part 4, a stainless steel part 6, and a filler material 8. While the titanium part 4 may be securely attached to stainless steel part 6 by any of several techniques that are known to those skilled in the art, brazing is the preferred method of attachment. In an alternative assembly, the stainless steel part 6 is attached to titanium part 4 by a threaded connection (not illustrated). Component assembly 2 is heated to a specific process temperature that is below the melting point of titanium part 4 or of the melting point of stainless steel part 6, for a specific period of time, at a pressure that is created by force 10, that is exerted to place filler material 8 in intimate contact with the titanium part 4 and stainless steel part 6.

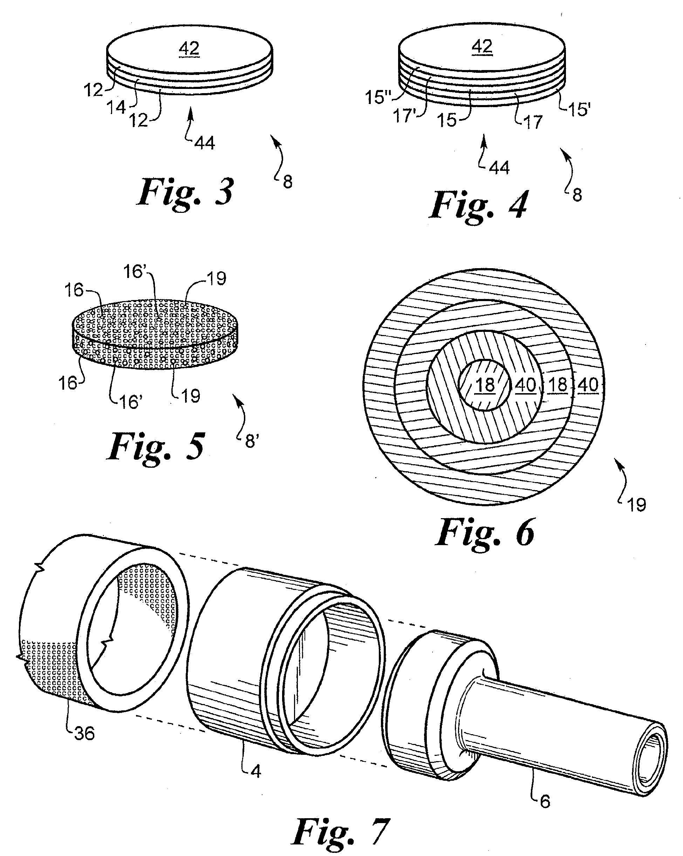

Filler material 8 is preferably a laminate metal foil having a thickness of approximately ten-thousandths (0.010) of an inch (250 micrometers) and more preferably less than 0.010 inches (250 micrometers) and even more preferably less than 0.001 inches (25 micrometers). In alternate embodiments, filler material 8 comprises substantially pure nickel, nickel alloys, or titanium-nickel alloys, where the substantially pure nickel is preferably greater than 98% nickel. Filler material 8 is selected from the group of materials that are compatible with the stainless steel chosen for stainless steel part 6 in that they wet the surface during the bonding process and enter into a diffusion process with the stainless steel part 6, thereby creating a strong bond joint during processing. Filler material 8 is further selected from the group of materials that are compatible with the titanium part 4. Filler material 8 forms a bond between titanium part 4 and stainless steel part 6 at the bonding temperature and pressure utilized during processing. The group of filler materials that are compatible with both the stainless steel part 6 and the titanium part 4 includes substantially pure titanium and nickel laminate compositions, preferably comprising filler materials of about 22% to 98% nickel and the balance titanium. In a preferred embodiment, **FIG. 3****,** filler material 8 is preferably comprised of alternating foil layers 12 and 14. Preferably, for example, as shown in **FIG. 3****,** a laminate stack of commercially pure nickel layer 12 on the top outer surface 42 and a similar nickel layer 12 on the bottom outer surface 44. Sandwiched between the nickel layers 12 is a titanium layer 14. The nickel layer 12 having at least 99.0% nickel and less than 1.0% of other elements (which can be considered substantially pure) with a thickness of greater than approximately 0.0003 inches (7.5 micrometers) and the titanium layer 14 comprised of commercially pure titanium foil having at least 99.0% titanium and less than 1.0% of other elements with a thickness of greater than approximately 0.0003 inches (7.5 micrometers).

In some embodiments for a living tissue implantable component assembly 2, the titanium part is preferably selected from the group of implantable grade titanium and titanium alloys, specifically, unalloyed titanium (CP grades 1-4), Ti6Al-4V ELI wrought, Ti-6Al-4V standard grade wrought, Ti-6Al-17Nb wrought, Ti-5Al-2.5Fe, CP and Ti-6Al-14Vstandard grade powders for coating implants, Ti-13Nb-13Zr wrought, and Ti-12Mo-6Zr-2Fe wrought.

The inventors prefer the term "laminated" versus other descriptive, but equally applicable, terms such as "layered", "clad", or "composite" material. The laminated filler material is not an "alloy" of nickel and titanium. An alloy, which is defined as a homogeneous mixture of two or more metals, where the atoms of one replace or occupy interstitial positions between the atoms of the other, of nickel and titanium, for example, does not demonstrate the depressed melting point that is available at a eutectic composition when nickel and titanium are in intimate contact. The laminate material supplies substantially pure nickel to initiate bonding with other metals, such as titanium or stainless steel, for example, at relatively low eutectic temperatures. For example, the lowest liquidus temperature (also referred to herein as the melting point) in the nickel-titanium phase diagram occurs at 28% by weight nickel and is 942°C. Therefore, the optimum braze temperature will be greater than this temperature.

In a further preferred embodiment, **FIG. 4****,** the metal foil layers 15, 15', and 15", which are comprised of nickel, are placed in laminated filler material 8 as the top outer surface 42 and as the bottom outer surface 44, thereby making the nickel available to react directly with the stainless steel part 6 and the titanium part 4. Alternating layers of inner mating foil layer 17 and 17', which are comprised of titanium, are placed between the metal foil layers 15, 15', and 15".

It is known to those skilled in the art that the total composition of a laminate stack of alternating nickel and titanium foil is controlled by the thickness of the foil layers, where the volume fraction of nickel and titanium is converted to weight percent by accounting for the density of the nickel and titanium. For example, to achieve a total laminate stack composition of a filler material 8 having a composition of 50 weight percent Ni and 50 weight percent Ti, where the density of nickel is 8.90 g/cc and of titanium is 4.51 g/cc, the thickness of the filler material 8 will be 33.6% Ni foil and 66.4% Ti foil.

Titanium part 4 may be comprised of a titanium alloy and is comprised of Ti-6Al-4V, i.e. an alloy of titanium with 6 weight percent aluminum and 4 weight percent vanadium, in a preferred embodiment. Stainless steel part 6 may be comprised of one of the corrosion resistant and/or implantable stainless steels, such as a 200, 300, or 400 series stainless steel (e.g. 304 stainless steel), and in a preferred embodiment stainless steel part 6 comprises 316L stainless steel. This configuration of components offers the advantage of being biocompatible and of being capable of forming hermetic seals.

In an alternate embodiment, rather than using filler material 8 as a foil, filler material 8 may be a thin coating that is applied to the bonding surface of either the titanium part 4 or stainless steel part 6 by any of a variety of chemical processes, such as electroless plating and electroplating, or by any of a variety of thermal processes, such as sputtering, evaporating, or ion beam enhanced deposition.

In another embodiment, filler material 8 is applied as a thin coating of metallic beads, metallic powder, or discrete particles. The coating may be applied in any of several methods known to those skilled in the art, such as painting, spraying, or dipping. The applied coating consists of discrete particles of nickel and of titanium that aid in bonding the stainless steel part 6 and the titanium part 4 during the braze process.

In a component assembly, a compact filler material 8', **FIG. 5****,** is comprised of a bonded compact of primary alloy particulate 16 and secondary alloy particulate 16', where primary alloy particulate 16 is preferably comprised of a nickel alloy and primary alloy particulate 16' preferably comprises a titanium alloy. The compact filler material 8' is formed by any of several techniques that are known to one skilled in the art, including cold pressing, warm pressing, slurry preparation, etc. The intimate mixture of primary alloy particulate 16 and secondary alloy particulate 16' bond together as well as react with the stainless steel part 6 and the titanium part 4 during the braze operation to yield a bonded component assembly 2.

Another alternate embodiment of forming a bonded component assembly 2 utilizes the compact filler material 8', as presented in **FIG. 5****,** that is comprised of layered discrete particle 19, preferably spheres, comprises a layered or laminated composition, as shown in **FIG. 6****.** In a preferred embodiment, layered discrete particle 19 comprises alternating layers of primary particle laminate layer 18 and secondary particle laminate layer 40, where primary particle laminate layer 18 preferably comprises a nickel alloy, preferably substantially pure nickel, and secondary particle laminate layer 40 comprises a titanium alloy, preferably substantially pure titanium. The overall bonding methods and processes are analogous to those employed for the several embodiments. The compact filler material 8' is formed either by conventional densification processes, such as cold pressing the particulate into a preform, **FIG. 5****,** or the compact filler material 8' is formed in situ between the parts being bonded by applying the particulate to the metal parts being bonded prior to assembling them for brazing.

The process steps that are employed to create component assembly 2 with a strong bond between titanium part 4 and stainless steel part 6 are schematically represented in **FIG. 2****.** First, the surfaces to be bonded are prepared in step 20 by machining to assure that they will intimately conform to each other during bonding. The surfaces may be smoothed and cleaned.

In step 22, component assembly 2 is prepared with filler material 8 between titanium part 4 and stainless steel part 6. In step 24, force 10 is applied to compress filler material 8 between titanium part 4 and stainless steel part 6. Force 10 is sufficient to create intimate contact between the parts. Force 10 is applied to assure that a bond is formed between titanium part 4 and stainless steel part 6, thus creating a hermetic seal between the two parts. It is preferred that the resulting pressure be greater than about five psi (34.4 kPa).

In step 26, the assembly to be heat processed is placed in a furnace in a non-reactive atmosphere, which is preferably vacuum, but which, in an alternative embodiment, can be any of several atmospheres that are known to those skilled in the art, such as argon, nitrogen or hydrogen. A non-reactive atmosphere is applied before the furnace is heated to the processing temperature in step 28. A preliminary holding temperature may be utilized to allow the thermal mass of the parts to achieve equilibrium before proceeding with heating. In a preferred embodiment, the vacuum is less than 10⁻⁵ torr (1.3 x 10⁻³ Pa), to assure that the filler material 8 and titanium part 4 do not oxidize. Component assembly 2 is held at the selected temperature, which is between approximately 940° and 1260°C, for approximately 5 to 60 minutes, while force 10 continues to be exerted on filler material 8. The exact time, temperature and pressure are variable with each other so as to achieve a strong bond between titanium part 4 and stainless steel part 6. For example, in a preferred embodiment, a 316L stainless steel part is bonded to a Ti-6Al-4V part in vacuum at 10⁻⁶ torr (1.3 x 10⁻⁴ Pa) at approximately 1000°C for 10 minutes with a pressure of about 50 psi (344 kPa) on a nickel-titanium foil of approximately 0.002 (50 micrometers) inches total thickness.

The furnace is cooled and component assembly 2 is cooled to room temperature in step 30. In optional step 32, component assembly 2 is cleaned by being placed in a bath, after thermal processing is complete, to assure removal of all nickel and nickel salts. This bath is preferably an acid bath that etches the exposed surfaces of component assembly 2. In a preferred embodiment, the bath is nitric acid. Removal of nickel and nickel salts in the etch bath insures that component assembly 2 is biocompatible. Nickel and nickel salts are detrimental to living animal tissue. It is preferred that all of the nickel that is introduced as filler material 8 is combined with the titanium and is chemically tied up by thermal processing to be unavailable as free nickel or as a nickel salt. Component assembly 2 is biocompatible after bonding and processing.

In another arrangement, component assembly 2 is either an electrical sensor or an electrical stimulator that is implanted in a human body, although it could equally well be implanted in any animal. It must survive long periods in the hostile environment of a living body, which is basically a warm saline solution. In another arrangement, component assembly 2 is either a sensor or stimulator comprised of a hollow ceramic tube 36, containing various electronic components, that is bonded to a titanium part 4. The titanium part 4 and the stainless steel part 6 comprising an electrode 56 for communicating electrical signals between living tissue and the bonded device 52. The component assembly must be watertight, resisting salt-water intrusion as well as growth of living tissue into the titanium-to-stainless steel braze joint. **FIG. 7** presents an exploded isometric view of a ceramic tube 36 that is bonded to a titanium part 4 and a stainless steel part 6. The stainless steel part 6 is designed to accept an electrically conductive wire 50, for transmission of electrical signals.

Further, component assembly 2 does not corrode while implanted in the body. The materials are chosen such that post-bonding they are not susceptible to corrosion either individually or in the as-bonded state. Component assembly 2 resists electrolytic corrosion as well as crevice corrosion, because of the materials selected for construction of component assembly 2.

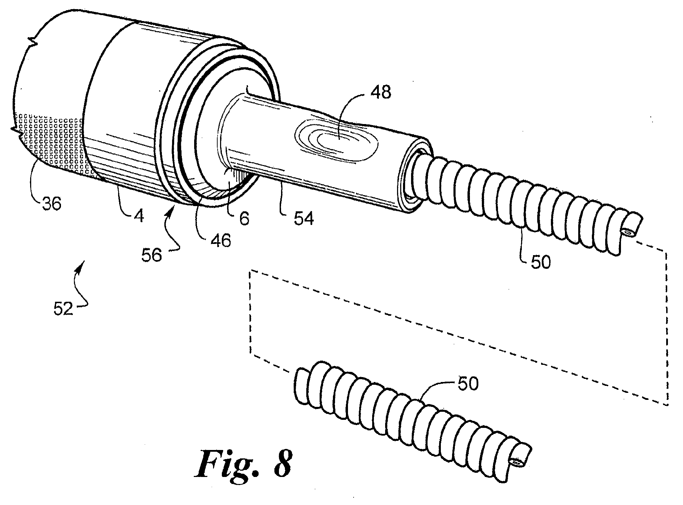

A bonded device 52 is presented in **FIG. 8** wherein a ceramic tube 36 is bonded to titanium part 4 which is bonded to stainless steel part 6 with a filler material 8 at braze joint 46. Stainless steel part 6 contains a receiver 54 into which a wire 50 is inserted and attached, preferably by crimping, such that crimp indentation 48 retains wire 50. The bonded device 52 provides good electrical conductivity via stainless steel part 6 connecting to wire 50. Stainless steel part 6 is brazed to titanium part 4 that is bonded by known methods to ceramic tube 36.

The bonded device 52 is preferably an implantable stimulator of muscle or nerve when implanted in living tissue, although it may operate as a sensor also. In a preferred embodiment, the device 52 has an outside diameter of 6 mm or less, enabling such a miniature device, which preferably has an overall length of less than about 60 mm, to be inserted in living tissue by injection, rather than by conventional surgical techniques. Ceramic tube 36 is an electrically insulating case, which typically is hollow and contains an electronics package and a power source, such as a battery, capacitor, magnetic field to electricity converter, and electrically conductive case ends described here as titanium part 4 and stainless steel part 6, forming an electrically conductive electrode 56 which conducts electrical signals from the stimulator and/or to a sensor, depending upon the design and function of that particular miniature bonded device 52. Bonded device 52 has at least one electrode, but preferably it has 2 to 8 or more electrodes, depending upon its particular design and function, although, for illustrative purposes, only one electrode is presented in **FIG 8****.**

Insulating case 4 contains the electronics, which may include a battery or other energy storage device and signal generating or receiving circuitry and is made of an electrically insulating ceramic material that is capable of being hermetically sealed and that is also biocompatible. The ceramic may be alumina, glass, titania, zirconia, stabilized-zirconia, partially-stabilized zirconia, tetragonal zirconia, magnesia-stabilized zirconia, ceria-stabilized zirconia, yttria-stabilized zirconia, or calcia-stabilized zirconia, and in a preferred embodiment, ceramic tube 36 is yttria-stabilized zirconia, although other ceramic materials may also be used. The ceramic tube 36 is preferably biocompatible as well as capable of being hermetically sealed, to prevent permeation of bodily fluids into the case.

The bond joints between the ceramic tube 36 and titanium part 4 and stainless steel 6 are preferably biocompatible, non-corrosive, and hermetic. To this end, it has been discovered that attaching an electrically insulating wire 50, which is suitable to conduct a signal to or from a remote location, to bonded device 52 presents a challenge when the wire was attached directly to titanium part 4 by, for example, by crimping or any of the methods known to one skilled in the art. The titanium part 4 develops an electrically insulating oxide layer that lowers the efficiency of the device and decreases useful life if an on-board battery is employed to power the device.

The solution is to employ stainless steel part 6 as the attachment electrode for wire 50. As presented in **FIG. 8****,** in another arrangement wire 50 is inserted into receiver 54, which is part of stainless steel part 6. The stainless steel does not corrode and provides a long-lived, low-resistance electrical contact with wire 50. The illustrated attachment method employs forming a crimp indentation 48 on receiver 54 that, in turn, secures electrically conductive wire 50 to stainless steel part 6.

In another arrangement, wire 50 is a highly conductive metal that is also benign in the body, such as MP35 alloy, although stainless steel, such as 316L, platinum, or an alloy of platinum-iridium may be employed. Preferably, the wire has a diameter of approximately 0.030 inches (750 micrometers). It is contained in a wire insulator to electrically isolate it from the body tissue and fluids and, in a preferred embodiment, the wire insulator is comprised of TEFLON, which is optionally coated with silicone.

Forming a strong, biocompatible bond between stainless steel part 6 and titanium part 4 was accomplished by forming braze joint 46 by utilizing a nickel-titanium or substantially pure nickel braze material.

These various embodiments are of devices and methods for connecting an electrically conductive wire 50 to a miniature implantable stimulator via a stainless steel to titanium electrode connector, in order to efficiently transmit or receive an electrical signal that is associated with the implantable stimulator.

Obviously, many modifications and variations of the present invention are possible in light of the above teachings. It is therefore to be understood that, within the scope of the appended claims, the invention may be practiced otherwise than as specifically described.

## Claims

1. A component assembly suitable for use in living tissue comprising:
a stainless steel part;
a titanium part; and
a filler material comprising at least one nickel foil layer and at least one titanium foil layer for bonding said stainless steel part to said titanium part.

2. The component assembly of claim 1, wherein said at least one nickel foil layer is adjacent said titanium part.

3. The component assembly of claim 1 or claim 2, wherein:
said filler material has a top and a bottom outer surface; and
said at least one nickel foil layer comprises the top and the bottom outer surfaces of said filler material.

4. The component assembly of claim 1, wherein:
said filler material has a top and a bottom outer surface; and
said at least one titanium foil layer comprises the top and the bottom outer surfaces of said filler material.

5. The component assembly of any one of the preceding claims, wherein said stainless steel part is selected from the group consisting of 200, 300, and 400 series stainless steel.

6. The component assembly of any one of claims 1 to 4, wherein said stainless steel part is comprised of 316L stainless steel.

7. The component assembly of any one of the preceding claims, wherein said titanium part is selected from the group consisting of titanium and titanium alloys.

8. The component assembly of any one of claims 1 to 6, wherein said titanium part is comprised of Ti-6Al-4V.

9. The component assembly of any one of the preceding claims, wherein said filler material reacts with and forms a bond between said titanium part and said stainless steel part.

10. The component assembly of any one of the preceding claims wherein:
said filler material has a thickness no greater than about 0.010 inches (250 micrometers); and
said component assembly being heated to a temperature that is less than the melting point of said titanium part or of said stainless steel part, but that is greater than the melting point of said filler material, thereby forming a bond.

11. The component assembly of any one of the preceding claims, wherein said at least one nickel foil layer and said at least one titanium foil layer are formed by a chemical process selected from the group consisting of electroless plating and electroplating, or by a thermal process selected from the group consisting of sputtering, evaporating, and ion beam enhanced deposition, or are formed from metallic particulate.

12. A method of bonding a stainless steel and titanium component assembly, comprising the steps of:
selecting a stainless steel part;
selecting a titanium part;
selecting a laminated filler material that is less than about 0.010 inches (250 micrometers) thick that is comprised of a 22% to 98% nickel portion and a remaining titanium portion, said laminated filler material comprising at least one nickel foil layer and at least one titanium foil layer,
selecting said laminated filler material having a melting point that is lower than the melting point of said titanium part and said stainless steel part;
positioning said filler material between said stainless steel part and said titanium part;
placing the assembly in a non-reactive atmosphere;
applying a force to said stainless steel part and said titanium part to place said filler material in compression, thereby creating intimate contact between said stainless steel part, said filler material, and said titanium part;
heating the assembly to a bonding temperature between said melting point of said laminated filler material and said melting point of said titanium part;
holding the assembly at said bonding temperature for a predetermined time to form a bond between said stainless steel part and said titanium part; and
cooling the assembly.

13. The method of claim 12 wherein said step of applying a force creates compression between about 5 and 50 psi (34.4 and 344 kPa).

14. The method of claim 12 wherein said step of applying a force creates compression between about 5 and 7 psi (34.4 and 48.3 kPa).

15. The method of any one of claims 12 to 14 wherein said step of selecting a stainless steel part is selecting from the group consisting of 200, 300, and 400 series stainless steel.

16. The method of any one of claims 12 to 15 wherein said step of selecting a titanium part is selecting from the group consisting of substantially pure titanium and its alloys.

17. The method of any one of claims 12 to 15 wherein said step of selecting a titanium part is selecting said part comprised of Ti-6Al-4V.

18. The method of any one of claims 12 to 17 further comprising the step of applying said filler material chemically or thermally or forming said filler material from metallic particulate.

19. The method of any one of claims 12 to 18 wherein the step of placing the assembly in a non-reactive atmosphere comprises placing the assembly in a vacuum less than 10⁻⁵ torr (1.3 x 10⁻³ Pa).

20. The method of any one of claims 12 to 19 wherein the step of placing the assembly in a non-reactive atmosphere comprises placing the assembly in argon gas.

21. The method of any one of claims 12 to 20 wherein said bonding temperature is between approximately 940° and 1260°C.

22. The method of any one of claims 12 to 21 wherein said predetermined time is between approximately 5 and 60 minutes.

23. The method of any one of claims 12 to 22 additionally comprising the step of cleaning said component assembly after bonding to remove elemental nickel and nickel salts.

24. The method of claim 23 additionally comprising the step of cleaning said component assembly after bonding by placing it in an acid bath.

25. A brazed component assembly comprising:
a stainless steel part;
a titanium part; and
a compact filler material comprising at least one set of metal composite particles, said at least one set of metal composite particle comprised of at least one primary particle laminate layer comprising a nickel alloy and at least one secondary sphere laminate layer comprising a titanium alloy, for bonding said stainless steel part to said titanium part.

26. The component assembly of claim 25, wherein said at least one primary particle laminate layer comprises substantially pure nickel.

27. The component assembly of claim 26, wherein said primary particle laminate layer comprising substantially pure nickel is at least about 99.0% nickel

28. The component assembly of any one of claims 25 to 27, wherein said at least one secondary particle laminate layer comprising substantially pure titanium.

29. The component assembly of claim 28, wherein said at least one secondary particle laminate layer comprising substantially pure titanium is at least about 99.0% titanium.

30. The component assembly of any one of claims 25 to 29, wherein said at least one primary particle laminate layer comprising a nickel alloy is an outer layer of said metal composite particle.

31. The component assembly of any one of claims 25 to 30, wherein said at least one primary particle laminate layer comprising a nickel alloy is about 22% to 50% by weight of said compact filler material.

32. The component assembly of any one of claims 25 to 31, wherein said stainless steel part is selected from the group consisting of 200, 300, and 400 series stainless steel.

33. The component assembly of any one of claims 25 to 32, wherein said stainless steel part is selected from the group consisting of implantable stainless steels.

34. The component assembly of any one of claims 25 to 31, wherein said stainless steel part comprises 316L stainless steel.

35. The component assembly of any one of claims 25 to 34, wherein said titanium part is selected from the group consisting of titanium and titanium alloys.

36. The component assembly of any one of claims 25 to 34, wherein said titanium part comprises Ti-6Al-4V.

37. The component assembly of any one of claims 25 to 36, wherein said compact filler material is formed in place between said stainless steel part and said titanium part.

## Patentansprüche

1. Komponentenanordnung, geeignet für den Einsatz in Lebendgewebe, umfassend:
einen Edelstahlteil;
einen Titanteil; und
ein Füllmaterial, das mindestens eine Nickelfolienschicht und mindestens eine Titanfolienschicht zum haftschlüssigen Verbinden des Edelstahlteils mit dem Titanteil umfasst.

2. Komponentenanordnung nach Anspruch 1, wobei die mindestens eine Nickelfolienschicht benachbart zum Titanteil ist;

3. Komponentenanordnung nach Anspruch 1 oder 2, wobei das Füllmaterial eine obere und eine untere Außenfläche aufweist und wobei die Nickelfolienschicht die obere und die untere Außenfläche des Füllmaterials umfasst.

4. Komponentenanordnung nach Anspruch 1, wobei
das Füllmaterial eine obere und eine untere Außenfläche aufweist und wobei
die Titanfolienschicht die obere und die untere Außenfläche des Füllmaterials umfasst.

5. Komponentenanordnung nach einem der vorangehenden Ansprüche, wobei der Edelstahlteil ausgewählt ist aus der Gruppe bestehend aus Edelstahl der Serie 200, 300 und 400.

6. Komponentenanordnung nach einem der Ansprüche 1 bis 4, wobei der Edelstahlteil aus 316L-Edelstahl besteht.

7. Komponentenanordnung nach einem der vorhergehenden Ansprüche, wobei der Titanteil ausgewählt ist aus der Gruppe bestehend aus Titan und Titanlegierungen.

8. Komponentenanordnung nach einem der Ansprüche 1 bis 6, wobei der Titanteil aus Ti-6Al-4V besteht.

9. Komponentenanordnung nach einem der vorhergehenden Ansprüche, wobei der Füllmaterial mit dem Titanteil und dem Edelstahlteil reagiert und zwischen diesen eine haftschlüssige Verbindung ausbildet.

10. Komponentenanordnung nach einem der vorhergehenden Ansprüche, wobei
das Füllmaterial eine Dicke von nicht mehr als ungefähr 0,010 Zoll (250 Mikrometer) aufweist; und wobei
die Komponentenanordnung auf eine Temperatur erhitzt wird, die niedriger als der Schmelzteil des Titanteils oder des Edelstahlteils, aber höher als der Schmelzpunkt des Füllmaterials ist, wodurch eine haftschlüssige Verbindung ausgebildet wird.

11. Komponentenanordnung nach einem der vorhergehenden Ansprüche, wobei die mindestens eine Nickelfolienschicht und die mindestens eine Titanfolienschicht durch einen chemischen Prozess, ausgewählt aus der Gruppe bestehend aus stromloser Plattierung und Elektroplattierung, oder durch einen thermischen Prozess, ausgewählt aus der Gruppe bestehend aus Zerstäubung, Verdampfung, durch Ionenstrahl verbesserte Beschichtung, oder aus Metallteilchen ausgebildet sind.

12. Verfahren zum haftschlüssigen Verbinden einer Edelstahl- und Titankomponentenanordnung, folgende Schritte umfassend:
Auswählen eines Edelstahlteils;
Auswählen eines Titanteils;
Auswählen eines laminierten Füllmaterials, das weniger als ungefähr 0,010 Zoll (250 Mikrometer) dick ist und zu 22 % bis 98 % aus Nickel und zum restlichen Prozentsatz aus Titan besteht, wobei das laminierte Füllmaterial mindestens eine Nickelfolienschicht und mindestens eine Titanfolienschicht umfasst,
Auswählen des laminierten Füllmaterials, das einen Schmelzpunkt aufweist, der niedriger ist als der Schmelzpunkt des Titanteils und des Edelstahlteils;
Positionieren des Füllmaterials zwischen dem Edelstahlteil und den Titanteil;
Platzieren der Anordnung in einer nichtreaktiven Atmosphäre;
Beaufschlagen einer Kraft auf den Edelstahlteil und den Titanteil, um das Füllmaterial zusammenzudrücken, wodurch ein enger Kontakt zwischen dem Edelstahlpart, dem Füllmaterial und dem Titanteil erzeugt wird;
Erhitzen der Anordnung auf eine Haftverbindungstemperatur zwischen dem Schmelzpunkt des laminierten Füllmaterials und dem Schmelzpunkt des Titanteils;
Halten der Anordnung bei der Haftverbindungstemperatur eine vorbestimmte Zeit lang, um eine haftschlüssige Verbindung zwischen dem Edelstahlteil und dem Titanteil auszubilden; und
Abkühlen der Anordnung.

13. Verfahren nach Anspruch 12, wobei der Schritt des Beaufschlagens einer Kraft eine Kompression zwischen 5 und 50 psi (34,4 und 344 kPa) erzeugt.

14. Verfahren nach Anspruch 12, wobei der Schritt des Beaufschlagens einer Kraft eine Kompression zwischen 5 und 7 psi (34,4 und 48,3 kPa) erzeugt.

15. Verfahren nach einem der Ansprüche 12 bis 14, wobei der Schritt des Auswählens eines Edelstahlteils das Auswählen aus der Gruppe bestehend aus Edelstahl der Serie 200, 300 und 400 ist.

16. Verfahren nach einem der Ansprüche 12 bis 15, wobei der Schritt des Auswählens eines Titanteils das Auswählen aus der Gruppe bestehend aus im Wesentlichen reinem Titan und seinen Legierungen ist.

17. Verfahren nach einem der Ansprüche 12 bis 15, wobei der Schritt des Auswählens eines Titanteils das Auswählen eines Teils, der aus Ti-6Al-4V besteht, ist.

18. Verfahren nach einem der Ansprüche 12 bis 17, ferner umfassend den Schritt des chemischen oder thermischen Auftragens des Füllmaterials oder Ausbilden desselben aus Metallteilchen.

19. Verfahren nach einem der Ansprüche 12 bis 18, wobei der Schritt des Platzierens der Anordnung in einer nichtreaktiven Atmosphäre das Platzieren der Anordnung in einem Vakuum von weniger als 10⁻⁵ torr (1,3 x 10⁻³ Pa).

20. Verfahren nach einem der Ansprüche 12 bis 19, wobei der Schritt des Platzierens der Anordnung in einer nichtreaktiven Atmosphäre das Platzieren der Anordnung in Argongas umfasst.

21. Verfahren nach einem der Ansprüche 12 bis 20, wobei die Haftverbindungstemperatur zwischen ungefähr 940° und 1260° liegt.

22. Verfahren nach einem der Ansprüche 12 bis 21, wobei die vorbestimmte Zeit zwischen ungefähr 5 und 60 Minuten liegt.

23. Verfahren nach einem der Ansprüche 12 bis 22, ferner umfassend den Schritt des Reinigens der Komponentenanordnung nach dem haftschlüssigen Verbinden, um Elementarnickel und Nickelsalze zu entfernen.

24. Verfahren nach Anspruch 23, ferner umfassend den Schritt des Reinigens der Komponentenanordnung nach dem haftschlüssigen Verbinden durch Platzieren der Anordnung in einem Säurebad.

25. Hartlötkomponentenanordnung, umfassend:
einen Edelstahlteil;
einen Titanteil; und
ein kompaktes Füllmaterial, das mindestens einen Satz von Metallverbundteilchen umfasst, wobei der mindestens eine Satz von Metallverbundteilchen aus mindestens einer primären Teilchenlaminatschicht, die eine Nickellegierung umfasst, und mindestens einer sekundären Teilchenlaminatschicht, die eine Titanlegierung umfasst, besteht, zum haftschlüssigen Verbinden des Edelstahlteils mit dem Titanteils.

26. Komponentenanordnung nach Anspruch 25, wobei die mindestens eine primäre Teilchenlaminatschicht im Wesentlichen reinen Nickel umfasst.

27. Komponentenanordnung nach Anspruch 26, wobei die mindestens eine primäre Teilchenlaminatschicht, die im Wesentlichen reinen Nickel umfasst, aus mindestens ungefähr 99,0 % Nickel besteht.

28. Komponentenanordnung nach einem der Ansprüche 25 bis 27, wobei die mindestens eine sekundäre Teilchenlaminatschicht im Wesentlichen reines Titan umfasst.

29. Komponentenanordnung nach Anspruch 28, wobei die mindestens eine sekundäre Teilchenlaminatschicht, die im Wesentlichen reines Titan umfasst, aus mindestens ungefähr 99,0 % Titan besteht.

30. Komponentenanordnung nach einem der Ansprüche 25 bis 29, wobei die mindestens eine primäre Teilchenlaminatschicht, die eine Nickellegierung umfasst, eine Außenschicht aus den Metallverbundteilchen ist.

31. Komponentenanordnung nach einem der Ansprüche 25 bis 30, wobei die mindestens eine primäre Teilchenlaminatschicht, die eine Nickellegierung umfasst, ungefähr 22 Gew.-% bis 50 Gew.-% des kompakten Füllmaterials ist.

32. Komponentenanordnung nach einem der Ansprüche 25 bis 31, wobei der Edelstahlteil ausgewählt ist aus der Gruppe bestehend aus Edelstahl der Serie 200, 300 und 400.

33. Komponentenanordnung nach einem der Ansprüche 25 bis 32, wobei der Edelstahlteil ausgewählt ist aus der Gruppe bestehend aus implantierbaren Edelstählen.

34. Komponentenanordnung nach einem der Ansprüche 25 bis 31, wobei der Edelstahlteil aus 316L-Edelstahl besteht.

35. Komponentenanordnung nach einem der Ansprüche 25 bis 34, wobei der Titanteil ausgewählt ist aus der Gruppe bestehend aus Titan und Titanlegierungen.

36. Komponentenanordnung nach einem der Ansprüche 25 bis 34, wobei der Titanteil aus Ti-6Al-4V besteht.

37. Komponentenanordnung nach einem der Ansprüche 25 bis 36, wobei das kompakte Füllmaterial zwischen dem Edelstahlteil und dem Titanteil vor Ort ausgebildet wird.

## Revendications

1. Assemblage de composants adapté à une utilisation dans un tissu vivant comprenant :
une pièce en acier inoxydable ;
une pièce en titane ; et
un matériau d'apport comprenant au moins une couche de feuille de nickel et au moins une couche de feuille de titane servant à relier ladite pièce en acier inoxydable à ladite pièce en titane.

2. Assemblage de composants selon la revendication 1, dans lequel ladite au moins une couche de feuille de nickel est adjacente à ladite pièce en titane.

3. Assemblage de composants selon la revendication 1 ou la revendication 2, dans lequel :
ledit matériau d'apport comporte une surface supérieure et une surface externe inférieure ; et
ladite au moins une couche de feuille de nickel comprend la surface supérieure et la surface externe inférieure dudit matériau d'apport.

4. Assemblage de composants selon la revendication 1, dans lequel :
ledit matériau d'apport comporte une surface supérieure et une surface externe inférieure ; et
ladite au moins une couche de feuille de titane comprend la surface supérieure et la surface externe inférieure dudit matériau d'apport.

5. Assemblage de composants selon l'une quelconque des revendications précédentes, dans lequel ladite pièce en acier inoxydable est choisie dans le groupe constitué de l'acier inoxydable des séries 200, 300 et 400.

6. Assemblage de composants selon l'une quelconque des revendications 1 à 4, dans lequel ladite pièce en acier inoxydable est composée d'acier inoxydable 316L.

7. Assemblage de composants selon l'une quelconque des revendications précédentes, dans lequel ladite pièce en titane est choisie dans le groupe constitué du titane et des alliages de titane.

8. Assemblage de composants selon l'une quelconque des revendications 1 à 6, dans lequel ladite pièce en titane est composée de Ti-6Al-4V.

9. Assemblage de composants selon l'une quelconque des revendications précédentes, dans lequel ledit matériau d'apport réagit avec ladite pièce en titane et ladite pièce en acier inoxydable et forme une liaison entre celles-ci.

10. Assemblage de composants selon l'une quelconque des revendications précédentes dans lequel :
ledit matériau d'apport a une épaisseur non supérieure à environ 0,010 pouce (250 micromètres) ; et
ledit assemblage de composants étant chauffé à une température qui est inférieure au point de fusion de ladite pièce en titane ou de ladite pièce en acier inoxydable, mais qui est supérieure au point de fusion dudit matériau d'apport, pour ainsi former une liaison.

11. Assemblage de composants selon l'une quelconque des revendications précédentes, dans lequel ladite au moins une couche de feuille de nickel et ladite au moins une couche de feuille de titane sont formées par un procédé chimique choisi dans le groupe constitué du placage anélectrolytique et du dépôt électrolytique, ou par un procédé thermique choisi dans le groupe constitué de la pulvérisation, de l'évaporation et du dépôt assisté par faisceaux d'ions, ou sont formées à partir de particules métalliques.

12. Procédé de liaison d'un assemblage de composants d'acier inoxydable et de titane, comprenant les étapes suivantes :
choix d'une pièce en acier inoxydable ;
choix d'une pièce en titane ;
choix d'un matériau d'apport stratifié dont l'épaisseur est inférieure à environ 0,010 pouce (250 micromètres) qui est composé d'une partie comprenant 22 % à 98 % de nickel et d'une partie restante constituée de titane, ledit matériau d'apport stratifié comprenant au moins une couche de feuille de nickel et au moins une couche de feuille de titane,
choix dudit matériau d'apport stratifié ayant un point de fusion qui est inférieur au point de fusion de ladite pièce en titane et de ladite pièce en acier inoxydable ;
positionnement dudit matériau d'apport entre ladite pièce en acier inoxydable et ladite pièce en titane ;
mise en place de l'assemblage dans une atmosphère non réactive ;
application d'une force sur ladite pièce en acier inoxydable et ladite pièce en titane pour placer ledit matériau d'apport en compression, en créant ainsi un contact intime entre ladite pièce en acier inoxydable, ledit matériau d'apport, et ladite pièce en titane ;
chauffage de l'assemblage à une température de liaison entre ledit point de fusion dudit matériau d'apport stratifié et ledit point de fusion de ladite pièce en titane ;
maintien de l'assemblage à ladite température de liaison pendant une durée prédéterminée pour former une liaison entre ladite pièce en acier inoxydable et ladite pièce en titane ; et
refroidissement de l'assemblage.

13. Procédé selon la revendication 12 dans lequel ladite étape d'application d'une force crée une compression entre environ 5 et 50 psi (34,4 et 344 kPa).

14. Procédé selon la revendication 12 dans lequel ladite étape d'application d'une force crée une compression entre environ 5 et 7 psi (34,4 et 48,3 kPa).

15. Procédé selon l'une quelconque des revendications 12 à 14 dans lequel ladite étape de choix d'une pièce en acier inoxydable est choisie dans le groupe constitué de l'acier inoxydable des séries 200, 300 et 400.

16. Procédé selon l'une quelconque des revendications 12 à 15 dans lequel ladite étape de choix d'une pièce en titane est choisie dans le groupe constitué du titane sensiblement pur et de ses alliages.

17. Procédé selon l'une quelconque des revendications 12 à 15 dans lequel ladite étape de choix d'une pièce en titane consiste à choisir ladite pièce composée de Ti-6Al-4V.

18. Procédé selon l'une quelconque des revendications 12 à 17 comprenant en outre l'étape d'application dudit matériau d'apport chimiquement ou thermiquement ou de formation dudit matériau d'apport à partir de particules métalliques.

19. Procédé selon l'une quelconque des revendications 12 à 18 dans lequel l'étape de mise en place de l'assemblage dans une atmosphère non réactive comprend la mise en place de l'assemblage dans un vide inférieur à 10⁻⁵ torr (1,3 x 10⁻³ Pa).

20. Procédé selon l'une quelconque des revendications 12 à 19 dans lequel l'étape de mise en place de l'assemblage dans une atmosphère non réactive comprend la mise en place de l'assemblage dans un gaz d'argon.

21. Procédé selon l'une quelconque des revendications 12 à 20 dans lequel ladite température de liaison se situe entre approximativement 940° et 1 260 °C.

22. Procédé selon l'une quelconque des revendications 12 à 21 dans lequel ladite durée prédéterminée se situe entre approximativement 5 et 60 minutes.

23. Procédé selon l'une quelconque des revendications 12 à 22 comprenant en outre l'étape de nettoyage dudit assemblage de composants après la liaison pour éliminer le nickel élémentaire et les sels de nickel.

24. Procédé selon la revendication 23 comprenant en outre l'étape de nettoyage dudit assemblage de composants après la liaison par sa mise en place dans un bain d'acide.

25. Assemblage de composants brasé comprenant :
une pièce en acier inoxydable ;
une pièce en titane ; et
un matériau d'apport compact comprenant au moins un ensemble de particules composites métalliques, ledit au moins un ensemble de particules composites métalliques étant composé d'au moins une couche stratifiée de particules primaires comprenant un alliage de nickel et au moins une couche stratifiée de sphères secondaires comprenant un alliage de titane, servant à relier ladite pièce en acier inoxydable à ladite pièce en titane.

26. Assemblage de composants selon la revendication 25, dans lequel ladite au moins une couche stratifiée de particules primaires comprend du nickel sensiblement pur.

27. Assemblage de composants selon la revendication 26, dans lequel ladite couche stratifiée de particules primaires comprenant du nickel sensiblement pur comprend au moins environ 99,0 % de nickel.

28. Assemblage de composants selon l'une quelconque des revendications 25 à 27, dans lequel ladite au moins une couche stratifiée de particules secondaires comprend du titane sensiblement pur.

29. Assemblage de composants selon la revendication 28, dans lequel ladite au moins une couche stratifiée de particules secondaires comprenant du titane sensiblement pur comprend au moins environ 99,0 % de titane.

30. Assemblage de composants selon l'une quelconque des revendications 25 à 29, dans lequel ladite au moins une couche stratifiée de particules primaires comprenant un alliage de nickel est une couche externe desdites particules composites métalliques.

31. Assemblage de composants selon l'une quelconque des revendications 25 à 30, dans lequel ladite au moins une couche stratifiée de particules primaires comprenant un alliage de nickel comprend environ 22 % à 50 % en poids dudit matériau d'apport compact.

32. Assemblage de composants selon l'une quelconque des revendications 25 à 31, dans lequel ladite pièce en acier inoxydable est choisie dans le groupe constitué de l'acier inoxydable des séries 200, 300 et 400.

33. Assemblage de composants selon l'une quelconque des revendications 25 à 32, dans lequel ladite pièce en acier inoxydable est choisie dans le groupe constitué des aciers inoxydables implantables.

34. Assemblage de composants selon l'une quelconque des revendications 25 à 31, dans lequel ladite pièce en acier inoxydable comprend de l'acier inoxydable 316L.

35. Assemblage de composants selon l'une quelconque des revendications 25 à 34, dans lequel ladite pièce en titane est choisie dans le groupe constitué du titane et des alliages de titane.

36. Assemblage de composants selon l'une quelconque des revendications 25 à 34, dans lequel ladite pièce en titane comprend du Ti-6Al-4V.

37. Assemblage de composants selon l'une quelconque des revendications 25 à 36, dans lequel ledit matériau d'apport compact est formé en place entre ladite pièce en acier inoxydable et ladite pièce en titane.
